Europäisches Patentamt

European Patent Office

Office européen des brevets

(19)

(11) Veröffentlichungsnummer: **0 324 361**
**A1**

## (12) EUROPÄISCHE PATENTANMELDUNG

(21) Anmeldenummer: **89100083.8**

(22) Anmeldetag: **04.01.89**

(51) Int. Cl.⁴: **A61M 15/06**

(30) Priorität: **11.01.88 DE 8800218 U**

(43) Veröffentlichungstag der Anmeldung:
**19.07.89 Patentblatt 89/29**

(84) Benannte Vertragsstaaten:
**AT BE CH DE ES FR GB GR IT LI LU NL SE**

(71) Anmelder: **BURGER SÖHNE AG BURG**
**Hauptstrasse 55**
**CH-5736 Burg(CH)**

(72) Erfinder: **Burger, Christian**
**Im Hof 99**
**CH-5736 Burg(CH)**

(74) Vertreter: **Bunke, Holger, Dr.rer.nat.**
**Dipl.-Chem. et al**
**Patentanwälte Prinz, Leiser, Bunke & Partner**
**Manzingerweg 7**
**D-8000 München 60(DE)**

(54) **Inhalator und dessen Verwendung.**

(57) Der erfindungsgemäße Inhalator zum Inhalieren von Dämpfen leicht flüchtiger Stoffe besteht im wesentlichen aus einem rohrförmigen Behälter (3), der mit gasdurchlässigen Stopfen (2, 4) verschlossen ist. Im Inneren (5) des Behälters (3) befinden sich Füllkörper (6), die leicht flüchtige, inhalierbare Stoffe enthalten. Der Inhalator wird vorzugsweise als eigenständiges Genußmittel, als Zigarettenersatz oder zur Pflege des Mund- und Rachenraums mittels der inhalierten Dämpfe verwendet.

EP 0 324 361 A1

## Inhalator und dessen Verwendung

Die Erfindung betrifft einen Inhalator zum Inhalieren von Dämpfen leicht flüchtiger Stoffe und dessen Verwendung, insbesondere zur Pflege des Mund- und Rachenraumes.

Inhalatoren werden zu medizinischen und nicht-medizinischen Zwecken seit langem angewandt. Im allgemeinen handelt es sich dabei um mehr oder weniger komplizierte Geräte, mit deren Hilfe leicht flüchtige Wirkstoffe meist aus wäßriger oder alkoholischer Lösung gasförmig ausgetrieben oder fein zerstäubt werden, so daß man die Dämpfe oder Aerosole über Mund oder Nase einatmen kann. Die Anwendung dieser komplizierten und teuren Apparate ist auf Kliniken, Arztpraxen, Heil- und Kuranstalten beschränkt.

Bei chronischen Erkrankungen des Hals- und Rachenraumes, bei reinen Vorsorgemaßnahmen und bei eher kosmetischen Anwendun gen, die man zu Hause oder unterwegs oder am Arbeitsplatz durchführen will, können solche Apparate nicht eingesetzt werden. Es bestand deshalb ein Bedürfnis nach der Bereitstellung eines einfachen und billigen Inhalators im Handtaschenformat, der überall hin mitgeführt werden kann und der außerdem eine äußere Form besitzt, die nicht sofort auf eine medizinische Behandlung schließen läßt.

Der Erfindung liegt die Aufgabe zugrunde, dieses Bedürfnis zu befriedigen.

Diese Aufgabe wird erfindungsgemäß durch die Bereitstellung eines Inhalators zum Inhalieren von Dämpfen leicht flüchtiger Stoffe gelöst, der gekennzeichnet ist durch einen rohrförmigen Behälter, der mit gasdurchlässigen Stopfen verschlossen ist, sowie durch im Inneren des Behälters befindliche Füllkörper, die leicht flüchtige, inhalierbare Stoffe enthalten.

Durch die rohrförmige Form des Inhalators, die an ein Schreibgerät, eine Zigarre, ein Zigarillo oder eine Zigarette erinnert, kann dieser jederzeit und überall hin mitgeführt werden, ebenso, wie z. B. ein Kugelschreiber oder ein Bleistift. Die Tatsache, daß der mit Füllkörpern gefüllte rohrförmige Behälter mit gasdurchlässigen Stopfen verschlossen ist, erlaubt es, an dem Röhrchen, wie an einer Zigarette, einem Zigarillo oder einer Zigarre, zu saugen und so die von dem durch das Röhrchen hindurchtretenden Luftstrom verflüchtigten bzw. mitgerissenen Dämpfe der in den Füllkörpern enthaltenen leicht flüchtigen Stoffe zu inhalieren.

Vorzugsweise ist der rohrförmige Behälter eine Zigarettenhülse, also ein ausgesprochen billiger Massenartikel, der es gestattet, den Inhalator nach einmaligem Gebrauch wegzuwerfen bzw. in den Hausmüll zu geben. Da vorzugsweise alle Bestandteile des Inhalators rückstandsfrei verbrannt werden können, wird die Umwelt hierdurch nicht belastet.

Die Stopfen, mit denen der rohrförmige Behälter, vorzugsweise an den beiden Enden, verschlossen ist, bestehen vorzugsweise aus Zellstoff oder Watte. Mindestens einer der beiden Stopfen kann aus einem handelsüblichen Zigarettenfilter bestehen.

Die Füllkörper bestehen vorzugsweise aus Mikrokapseln, die die leicht flüchtigen, inhalierbaren Stoffe enthalten, mit ihnen getränkt oder imprägniert sind. Die Füllkörper enthalten vorzugsweise ätherische Öle oder sonstige leicht flüchtige, inhalierbare Aromen, Duft- oder Geschmacksstoffe, meist in Form pflanzlicher Öle oder natürlicher oder naturidentischer Aromen. Es können jedoch auch synthetisch hergestellte leicht flüchtige Stoffe verwendet werden, soweit sie pharmakologisch unbedenklich sind.

Beispiele für solche leicht flüchtigen, inhalierbaren Stoffe sind Pfefferminz- und Eukalyptusöl, Öle des australischen Teebaums (tea tree oil), japanisches Heilpflanzenöl, Kamillenöl, Latschenkiefernöl, ätherische Öle, insbesondere von Zitrusfrüchten und -pflanzen, natürliches oder naturidentisches Apfelaroma und dergleichen.

Der erfindungsgemäße Inhalator kann zum Schutz der inhalierbaren Stoffe vor vorzeitiger Verflüchtigung einzeln oder zu mehreren in Aluminium- und/cder Kunststoffolie verpackt und durch Versiegelung luftdicht verschlossen sein.

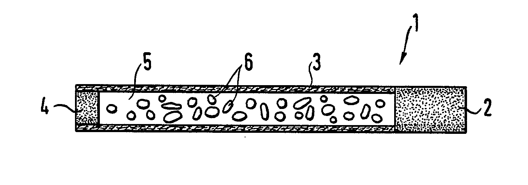

Die Erfindung wird anhand der Zeichnung weiter erläutert:

Bei dem gezeichneten Ausführungsbeispiel besitzt der Inhalator 1 die äußere Form einer Zigarette. Er besteht aus einem rohrförmigen Behälter, nämlich einer Zigarettenhülse 3, deren eines Ende mit einem Zigarettenfilter 2 und deren anderes Ende mit einem Stopfen 4 aus Zellstoff oder Watte verschlossen ist. Im Innenraum 5 der Zigarettenhülse 3 befinden sich Füllkörper 6, die leicht flüchtige, inhalierbare Stoffe, z. B. Pfefferminz- und Eukalyptusöl, enthalten.

Beim bestimmungsgemäßen Gebrauch wird der Inhalator wie eine Zigarette "geraucht", ohne jedoch angezündet zu werden, d. h. der Anwender zieht an einem der beiden Stopfen, vorzugsweise an dem Zigarettenfilter 2, und saugt so einen Luftstrom an, der die in den Füllkörpern 6 enthaltenen leicht flüchtigen Stoffe verflüchtigt bzw. mitreißt. Auf diese Weise gelangen die Dämpfe der leicht flüchtigen Stoffe in den Mund- und Rachenraum.

Der Innenraum 5 des rohrförmigen Behälters 3 muß nicht vollständig mit Füllkörpern 6 gefüllt sein. Je nach dem zu inhalierenden Stoff, der zu inhalierenden Konzentration oder der gewünschten Geschmacksrichtung kann auch eine Teilfüllung ausreichend sein. Die Füllkörper 6 können alle mit dem gleichen leicht flüchtigen Stoff, aber auch mit verschiedenen Stoffen getränkt oder imprägniert sein. Die Verschlußstopfen 2, 4 müssen nicht an den Enden des rohrförmigen Behälters 3 angebracht sein, vielmehr kann der rohrförmige Behälter über die Stopfen hinausragen, wodurch ein Hohlmundstück entsteht, so, wie es etwa bei den in Osteuropa verbreiteten Machorka-Zigaretten der Fall ist.

Der erfindungsgemäße Inhalator kann sowohl zu medizinischen Zwecken als auch zu eher kosmetischen Zwecken, zur Pflege des Mund- und Rachenraums, zur Verhinderung von Mundgeruch, zur Beseitigung des Tabakgeruchs nach dem Rauchen oder auch zur Abgewöhnung des Rauchens, als Zigarettenersatz oder als eigenständiges, eine Alternative zu Tabakwaren darstellendes Genußmittel verwendet werden.

## Ansprüche

1. Inhalator zum Inhalieren von Dämpfen leicht flüchtiger Stoffe, gekennzeichnet durch einen rohrförmigen Behälter (3), der mit gasdurchlässigen Stopfen (2, 4) verschlossen ist, sowie durch im Inneren (5) des Behälters (3) befindliche Füllkörper (6), die leicht flüchtige, inhalierbare Stoffe enthalten.

2. Inhalator nach Anspruch dadurch gekennzeichnet, daß der rohrförmige Behälter (3) eine Zigarettenhülse ist.

3. Inhalator nach Anspruch 1 oder 2, dadurch gekennzeichnet, daß die Stopfen (2, 4) aus Zellstoff oder Watte bestehen.

4. Inhalator nach einem der Ansprüche 1 bis 3, dadurch gekennzeichnet, daß die Füllkörper (6) aus Mikrokapseln bestehen.

5. Inhalator nach einem der Ansprüche 1 bis 4, dadurch gekennzeichnet, daß die Füllkörper (6) ätherische Öle enthalten.

6. Inhalator nach einem der Ansprüche 1 bis 4, dadurch gekennzeichnet, daß die Füllkörper (6) leicht flüchtige, inhalierbare Aromen, Duft- oder Geschmacksstoffe enthalten.

7. Inhalator nach Anspruch 6, dadurch gekennzeichnet, daß die Füllkörper (6) natürliches oder naturidentisches Apfelaroma enthalten.

8. Inhalator nach einem der Ansprüche 1 bis 7, dadurch gekennzeichnet, daß er in eine versiegelte Aluminiumfolienpakkung luftdicht eingeschlossen ist.

9. Verwendung des Inhalators gemäß einem der Ansprüche 1 bis 8 zur Pflege des Mund- und Rachenraums mittels der inhalierten Dämpfe.

10. Verwendung des Inhalators gemäß einem der Ansprüche 1 bis 8 als Genußmittel und Zigarettenersatz.

**Europäisches Patentamt**

# EUROPÄISCHER RECHERCHENBERICHT

Nummer der Anmeldung

| | EINSCHLÄGIGE DOKUMENTE | | EP 89100083.8 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | <u>US - A - 533 880</u> (FORNE) | 1,3 | A 61 M 15/06 |
| Y | * Fig. 1,2; Seite 1, Zeilen 9-12, 18-28; Seite 2, Zeilen 23-32 * | 2,5-7, 9,10 | |
| | -- | | |
| Y | <u>DE - A - 2 149 190</u> (WILMS FLOET'S) | 2,5-7, 9,10 | |
| | * Fig. 1; Seiten 1-3 * | | |
| | -- | | |
| X | <u>AT - B - 45 100</u> (G. KRAUSE) | 1,3,9, 10 | |
| | * Gesamt * | | |
| | -- | | |
| A | <u>US - A - 2 603 215</u> (P. ARNOW) | 1,3 | |
| | * Fig. 3; Spalte 2, Zeilen 3-25 * | | |
| | ---- | | RECHERCHIERTE SACHGEBIETE (Int. Cl.4) |
| | | | A 61 M 15/00 |

Der vorliegende Recherchenbericht wurde für alle Patentansprüche erstellt

| Recherchenort | Abschlußdatum der Recherche | Prüfer |
|---|---|---|
| WIEN | 28-04-1989 | LUDWIG |